# EUROPEAN PATENT APPLICATION

(11) **EP 3 108 874 A1**
(43) Date of publication of application: **28.12.2016**
(21) Application number: 15173997.6
(22) Date of filing: 26.06.2015
(51) Int. Cl.: A61K 9/00

(54) **OPHTHALMOLOGIC PHARMACEUTICAL COMPOSITION**

(71) Applicant: TRB Chemedica AG, 85540 Haar/München (DE)
(72) Inventor: Kalkbrenner, Hans, 85591 Vaterstetten (DE)
(74) Representative: Viering, Jentschura & Partner mbB Patent- und Rechtsanwälte

(57) **Abstract**

The present invention relates to pharmaceutical compositions for the treatment or prevention of ophthalmologic diseases or disorders, wherein the pharmaceutical composition comprises a solid carrier in form of a non-woven or woven made of water-soluble fibers impregnated with at least one therapeutically active agent, wherein the solid carrier dissolves upon contact with the eye. Also encompassed are the use of such composition for the treatment or prevention of ophthalmologic conditions and the use of the non-wovens/wovens described herein as carriers for at least one therapeutically active agent in an ophthalmologic pharmaceutical composition.

## Description

### BACKGROUND OF THE INVENTION

In pharmaceutical applications it is essential that the active ingredients such as medicaments are applied to a specific site or via a specific route to achieve the desired effect. Moreover, the selection of the dosage regimen is of critical importance to ensure that the active ingredient is administered in the correct amount. Due to the uniqueness of eyes in terms of anatomic structure and physiology, delivering drugs to the eyes for the treatment of ophthalmologic disorders and diseases has been a challenge for a long time.

Currently, essentially all of the ophthalmologic drugs are manufactured in form of eye drops. However, this administration form has drawbacks in that it is difficult to ensure that the therapeutic agent(s) contained therein are retained on the cornea for a sufficient time and uptake and efficiency is influenced by a variety of factors, such as conjunctival blood flow, lymphatic clearance and tear dilution.

Another major problem is that eye-drops are usually self-administered by the patient. While this is convenient in that it allows the use at home, it is disadvantageous in that administering the correct dosage, i.e. the correct number of drops, may be difficult and that self-administration may in itself be a problem for patients, in particular the elderly. Thus, the eye is at risk of receiving a massive and unpredictable dose of medication or none at all.

Hence, there exists still need in the art for alternative dosage forms of ophthalmologic medications that overcome at least some of the drawbacks of existing dosage forms.

### SUMMARY OF THE INVENTION

The present invention is based on the unexpected finding that pharmaceutical compositions comprising a solid carrier in form of water-soluble fibers, which readily dissolve and thereby release a therapeutically active agent upon contact with the eye, are ideally suited for delivering specific doses of said therapeutically active agent to the eye. This is due to the fact that the administration of such a solid carrier is comparably simple and as the solid carrier only dissolves and releases the therapeutically active agent once it gets into contact with the eye, it can be ensured that the correct amount is delivered.

Hence, these novel pharmaceutical compositions are ideally suited for all kinds of applications especially those where patients administer drugs without supervision by a medical professional.

Thus, in a first aspect the present invention relates to a pharmaceutical composition for use in the treatment or prevention of ophthalmologic diseases or disorders, wherein the pharmaceutical composition comprises a solid carrier in form of a non-woven or woven made of water-soluble fibers impregnated with at least one therapeutically active agent and wherein the solid carrier dissolves upon contact with the eye.

In various embodiments, the solid carrier dissolves upon contact with the eye within a time period of up to 10 seconds.

In various embodiments, the water-soluble fibers comprise or consist of nanofibers.

In various embodiments the solid carrier dissolves and the water-soluble fibers completely dissolve upon contact with the eye within a time period of up to 1 second.

The nanofibers may comprise or consist of hyaluronan (hyaluronic acid) or a pharmaceutically acceptable salt thereof. In various embodiments, the nanofibers comprise 40 to 95 wt.-% hyaluronan and 5 to 60 wt.-% of at least one water-soluble hyaluronan derivative selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof. The salt of hyaluronan or hyaluronan derivative used may be an alkali metal salt, preferably the sodium salt.

To facilitate administration and handling the solid carrier may have the form of a strip and may be attached to a handle or tip.

In one embodiment the at least one therapeutically active agent is selected from the prostaglandin and prostaglandin analogues Latanoprost, Dorzolamid, Bimatoprost, Tafluprost, Travoprost and/or the carbonic anhydrase inhibitor Brinzolamid and/or the imidazolin Brimonidin and/or the beta-blocker Timolol and/or the antibiotics, Ofloxacin, Gentamicin, Dexamthason, Neomycin, and/or the anti-histamines Azelastin, Levocabastin, Ketotifen, and/or the alpha antagonist Tetryzolin and/or the antiseptic Bibrocathol and/or the anti-inflammatory drugs Prednisolon, Cromoglicic acid, Diclofenac, Ketorolac and/or Carbomer and Povidon.

In various embodiments, the ophthalmologic disease or disorder is selected from the group consisting of glaucoma, dry eye syndrome, macular degeneration, infection, inflammation and allergy.

In a further aspect, the invention relates to an ophthalmologic pharmaceutical composition comprising a solid carrier in form of a non-woven or woven made of water-soluble fibers impregnated with at least one therapeutically active agent, wherein the water-soluble fibers comprise or consist of nanofibers made of hyaluronan or a pharmaceutically acceptable salt thereof and wherein the solid carrier dissolves upon contact with the eye.

In various embodiments, the nanofibers comprise 40 to 95 wt.-% hyaluronan and 5 to 60 wt.-% of at least one water-soluble hyaluronan derivative selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salt thereof. The salt of hyaluronan or hyaluronan derivative used may be an alkali metal salt, preferably the sodium salt.

In a still further aspect, the present invention also covers the use of a non-woven or woven made of water-soluble fibers comprising or consisting of nanofibers made of hyaluronan or a pharmaceutically acceptable salt thereof as a solid carrier for at least one therapeutically active agent in an ophthalmologic pharmaceutical composition, wherein the non-woven or woven completely dissolves upon contact with the eye.

### BRIEF DESCRIPTION OF THE DRAWINGS

The invention will be better understood with reference to the detailed description when considered in conjunction with the accompanying drawings.

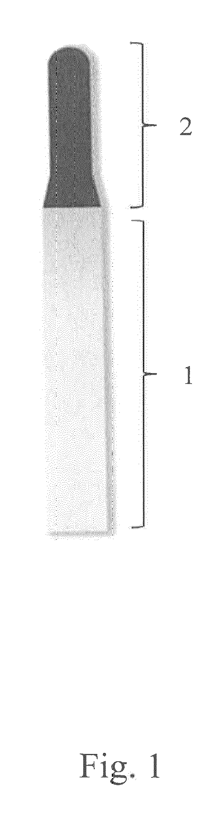

In Figure 1 an embodiment where the solid carrier is attached to a handle is schematically depicted. In detail, the solid carrier (1) is attached to a handle (2), which can be disposable or re-usable.

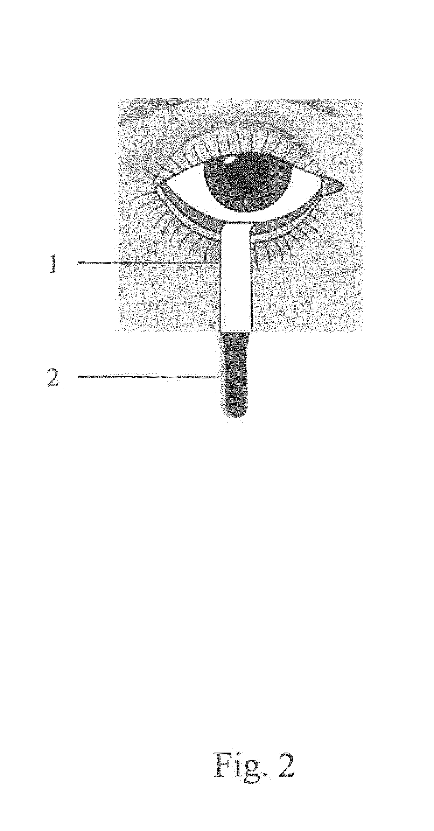

In Figure 2 it is schematically depicted how the patient can apply the solid carrier to the eye without touching it via using the handle (2). Upon contact with the aqueous tear fluid in the eye the solid carrier dissolves and releases the therapeutically active agent.

### DETAILED DESCRIPTION OF THE INVENTION

The present invention is based on the surprising finding that eye medication may be readily administered without the risk of spilling or overdosing by use of a solid administration form, namely a solid carrier made of fibers in form or a non-woven or woven fabric. The fibers readily and rapidly dissolve upon contact with an aqueous medium, such as the tear fluid of the eye, and release an active agent with which they have been impregnated during manufacture. The application of the solid carrier, which is typically in form of a strip, is comparably easy and due to the rapid dissolution kinetics of the fibers surprisingly comfortable. This behaviour makes the solid carrier ideally suited for delivering specific doses of said active agent to the eye. The fact that the solid carrier only dissolves once it comes into contact with the moist surface of the eye, prevents premature release of the active agent and ensures correct dosing.

As described above, the present invention is in a first aspect directed to a pharmaceutical composition for use in the treatment of ophthalmologic diseases or disorders, wherein the pharmaceutical composition comprises a solid carrier in form of a non-woven or woven made of water-soluble fibers impregnated with at least one therapeutically active agent and wherein the solid carrier dissolves upon contact with the eye.

The term "pharmaceutical composition" as used herein refers to a composition that is made such that it is suitable for administration to humans, e.g., it is made under good manufacturing practice (GMP) conditions. The composition is in solid form and may have the form of a strip. Typically, the composition is made of the solid carrier material, which is impregnated with the active agent and optionally all other ingredients. Accordingly, the composition may, in addition to the solid carrier and the active agent, contain additional pharmaceutically acceptable excipients, such as, without limitation, surfactants, stabilizers, bulking agents, buffering substances, auxiliaries, solubilizers, binders, and chelating agents.

The diluents may, for example, comprise propylene glycol and its derivates, glycerol and its derivatives, cellulose derivatives, sucrose and its derivatives, fatty alcohols, fatty acids and its esters.

The auxiliaries may be selected from the group consisting of antioxidants, surfactants, preservatives, natural or synthetic oil components, alcohols, thickening agents, vitamins and inorganic salts or combinations thereof. The inorganic salts include, but are not limited to sodium chloride, calcium chloride, magnesium chloride, and potassium chloride.

In one embodiment, the pharmaceutical composition may comprise an antioxidant, such as a tocopherol, tocotrienol, tocomonoenol or marine tocopherol (MDT). The antioxidant may thus be selected from the group consisting of alpha-, beta-, gamma-, delta- tocopherol, alpha-, beta-, gamma-, delta-tocotrienol, alpha-, beta-, gamma-, delta- tocomonoenol, alpha-, beta-, gamma-, and delta-MDT and mixtures thereof.

In one embodiment, the pharmaceutical composition comprises a preservative. Useful preservatives are those that are non-irritating to the skin or the eye and which are compatible with the components of the pharmaceutical composition. Suitable preservatives include but are not limited to sorbic acid and ethylenediamine tetraacetic acid (EDTA). The preservative may be used in a biostatic amount that prevents contamination of the composition. The preservative may include polyhexanide.

In an alternative embodiment the pharmaceutical composition does not comprise a preservative. In such an embodiment it is preferred that the pharmaceutical composition is supplied in sterile packaging, e.g. in sterile pouches or blisters. Sterilization can be carried out, e.g.by moist heat, ethylene oxide (ETO) or gamma irradiation.

If the pharmaceutical composition includes surfactants, these are preferably non-ionic. Suitable examples include polysorbate, block copolymers of ethylene oxide and propylene oxide (Pluronic®), polyethoxylated castor oil (Cremophor®), crosslinked copolymers of acrylic acid, and C10-C30 alkyl acrylate (Pemulen®).

Buffering substances that may be used include, but are not limited to Tris (Tris(hydroxymethyl)aminomethane), NaOH, histidine, tricine, lysine, glycine and serine adjusted to the correct pH with an acidic component with low ionic force.

The pharmaceutical composition may also comprise further auxiliaries such as hypromellose (hydroxypropylmethylcellulose), chitosan, lubricin, xanthan gum, parabens, benzalkonium chloride, polyhexamethylene biguanide, arginine, glycine, heparin, sodium pentosan polysuifate and/or lysine.

The pharmaceutical composition may, once dissolved in water at 20°C have a pH in the range of from about 6 to about 8, about 7 or about 7.3.

The term "pharmaceutically acceptable" as used herein refers to a substance that neither causes unacceptable loss of pharmacological activity of the active agent nor has unacceptable adverse side effects.

As used herein, the term "active agent" or "active ingredient" refers to a substance intended to be delivered, i.e., the substance being capable of achieving a desired action or effect. Such substances include, but are not limited to, drugs, diagnostic agents, cosmetic agents, nutritional supplements and mixtures thereof. The desired action or effect may include but is not limited to having a prophylactic effect on the organism and preventing an undesired biological effect such as preventing an infection, alleviating a condition caused by a disease, for example, alleviating pain or inflammation caused as a result of disease, and/or either alleviating, reducing, or completely eliminating a disease from the organism. The effect may be local, or it may be systemic. In accordance therewith, "therapeutically active agent" refers to a substance that has a therapeutic effect on the treated organism. The treated organisms are typically mammals, preferably humans.

Non-limiting examples of active ingredients are antibiotics, antifungals, analgesics, anaesthetics, anti-allergics, anti- inflammatory agents, and agents to treat glaucoma, e.g. Prostaglandin analogs such as Xalatan®, Lumigan©, and Travatan Z®, beta blockers such as Timolol, alpha agonists such as Alphagan®P and iopidine® as well as carbonic anhydrase inhibitors.

The active ingredient is present in the pharmaceutical composition in an effective amount.

As used herein, "effective amount" means the amount of active ingredient that is sufficient to provide the desired local or systemic effect and performance at a reasonable risk/benefit ratio.

As used herein the term "solid carrier" refers to a pharmaceutical acceptable substance that delivers the active ingredient to the site of treatment. The carrier used in accordance with the invention is solid at standard pressure and temperature (20°C, 1013 mbar) and is provided in form of fibers that form a non-woven or woven fabric. The fibers are water-soluble in that they readily dissolve upon contact with an aqueous solution. Preferably the fibers completely dissolve upon contact with an aqueous solution.

As used herein the term "nonwoven" refers to a web that has a structure of individual fibers or threads that are interlaid, but not in any regular, repeating manner. Non-woven webs may be formed by a variety of processes such as, for example, meltblowing processes, spunbonding process, bonded carded web processes and in relation to nanofibers by electrospinning, electroblowing and electrospraying.

Similarly, the term "woven" refers to a fabric formed of individual fibers or threads that are interlaid in a regular repeating manner.

As used herein the term "dissolve" refers to the feature that the solid carrier disintegrates by dissolution in a solvent to such an extent that it is no longer felt or noticed by the subject to that it was administered. This involves that the water-soluble fibers separate and/or dissolve such that the carrier loses its structural integrity and is no longer perceptible as a foreign (solid) object by the subject. Preferably 50% or more, more preferably 70% or more, most preferably 90% or more of the fibers are hydrated, moisturized or dissolved when the solid carrier is dissolved. Dissolution may, for example, be measured by contacting the solid carrier with a solvent for a predetermined amount of time, separating the solvent and the solid carrier, for example by filtration, and determining the amount of solved fiber material in the filtrate or (for example gravimetrically) determining the remaining amount of solids retained by the filter. Alternatively, dissolution can be monitored by measuring turbity of a solvent/solid carrier mixture over time.

Accordingly, as used herein, the terms "dissolves" and "disintegrate" are used interchangeably and refer to the process, in which the internal structure of the solid carrier disassembles.

In various embodiments the solid carrier dissolves within a time period of up to 10 seconds, preferably up to 1 second. This is advantageous because due to the fast disintegration of the solid carrier the active agent of the pharmaceutical composition can be easily self-administered even by untrained patients or elder patients with slight tremors, who would usually have difficulties applying eye medications such as eye drops.

In especially preferred embodiments, the solid carrier dissolves immediately upon contact with an aqueous medium, such as the tear fluid of the eye, i.e. the disintegration time is up to 1 second, preferably up to 0.5, 0.1, 0.05, 0.02 or 0.01 seconds.

"Completely dissolved", as used herein, means that the solid carrier disintegrates and that also the water-soluble fibers dissolve such that the individual molecules making up the fiber become hydrated, e.g. in case the water-soluble fibers comprise or consist of hyaluronan they disintegrate to hyaluronan molecules. In various embodiments, the complete dissolution entails that 50% or more, more preferably 70% or more, most preferably 90% or more of the water-soluble fibers are disassembled into individual molecules.

In various embodiments the water-soluble fibers completely dissolve within a time period of up to 10 seconds, preferably up to 1 second. This is advantageous because due to the fast disintegration of the water-soluble fibers in turn also the solid carrier may dissolve even faster.

In especially preferred embodiments, the water-soluble fibres dissolves immediately upon contact with an aqueous medium, such as the tear fluid of the eye, i.e. the disintegration time is up to 1 second, preferably up to 0.5, 0.1, 0.05, 0.02 or 0.01 seconds.

In various embodiments the solid carrier dissolves and the water-soluble fibers completely dissolve immediately upon contact with an aqueous medium, such as the tear fluid of the eye, i.e. the disintegration time of both the solid carrier and the water-soluble fibers is up to 1 second, preferably up to 0.5, 0.1, 0.05, 0.02 or 0.01 seconds.

Such embodiments are especially preferred, since application of the active agent to the eye is almost instantaneous and does not cause any pain, irritation or discomfort for the patient. In other words, as soon as the person receiving the pharmaceutical composition blinks his eye(s) the active ingredient is already administered. This is especially advantageous for people experiencing difficulties in squeezing the vials of conventional eye drops or for those with tremors such as patients with weak hands and/or with neurodegenerative disorders such as Parkinson's disease.

Moreover, the pharmaceutical composition is advantageous as it allows a definite feedback about whether or not the active ingredient was applied to the eye. If administration has taken place the solid carrier is dissolved. If, for instance a person with a tremor, has missed the eye, the solid carrier is still perceptible and hence the patient may retry administration.

Thus, a specific and well-defined dose of an active ingredient may be delivered to the eye.

To achieve the desired dissolution kinetics upon contact with minute amounts of water, such as the moist surface of the cornea of the eye, the fibers are preferably nanofibers. As used herein the term "nanofiber" refers to fibers with a thickness, i.e., a diameter, of 50-1200 nm.

The nanofibers can for example be ordered in form of crosswire, in a random manner or lengthwise. Depending on the way the nanofibers are ordered the textile strength of a nanofiber nonwoven can range from 2000-14000 MPa.

The advantages of nanofibers are small fiber diameter, very large specific surface and a high amount of small inter-fibre pores. This leads to a high reactivity of the nanofibers due to their small dimension resulting in fast release and short diffusion pathways of incorporated active ingredients and in a fast degradation of the solid nanofiber carrier.

In various embodiments, the release profile of the active ingredient may be changed by altering the structure of the nanofibrous material.

In a preferred embodiment the solid carrier is a water-soluble nonwoven that consists of nanofibers.

In various embodiments, the nanofiber nonwoven has a weight from 0.5-200 g/m².

While the solid carrier can be provided in various sizes and shapes, selected based on the specific application, it typically has the form of a strip of a dimension that make it suitable for application to the eye. Such a strip is, in various embodiments, of roughly rectangular shape with a length of 1 to 6 cm, a breadth of 1 to 10 mm, and a thickness of up to 1 to 1000 µm. The solid carrier retains the active ingredient until it disintegrates upon contact with an aqueous solution, i.e. the tear fluid in the eye.

A solid carrier in form of a water-soluble nonwoven and a solid carrier comprising nanofibers, respectively, have the advantage that the solid carrier can dissolve very rapidly in a matter of seconds. This effect is particularly pronounced, if the solid water-soluble nonwoven carrier consists of nanofibers. Thus, a possibly discomfort experienced by the patient upon applying the pharmaceutical composition ceases very quickly. In such a scenario the initial discomfort then merely ensures the patient that the pharmaceutical composition was applied correctly.

In a preferred embodiment, the water-soluble fibers, preferably nanofibers, comprise or consist of hyaluronan.

The terms "hyaluronan" and "hyaluronic acid" and "HA" are used interchangeably herein and all relate to an anionic, nonsulfated glycosaminoglycan distributed widely throughout connective, epithelial, and neural tissues that is made of D-glucuronic acid and D-N-acetylglucosamine monomers linked via alternating β-1,4 and β-1,3 glycosidic bonds. It generally is a polymer of formula (I):

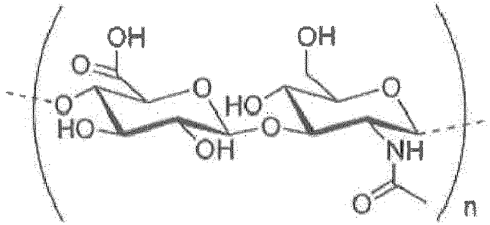

It is unique among glycosaminoglycans in that it is nonsulfated, forms in the plasma membrane instead of the Golgi, and can be very large, with its molecular weight often reaching the millions. One of the chief components of the extracellular matrix, hyaluronan contributes significantly to cell proliferation and migration.

Hyaluronan can be 25,000 disaccharide repeats in length, i.e. n in Formula (I) can be up to 25,000. Polymers of hyaluronan can range in size from 5,000 to 20,000,000 Da *in vivo.* The average molecular weight in human synovial fluid is 3-4 million Da, and hyaluronan purified from human umbilical cord is 3,140,000 Da.

In various embodiments the nanofibers further comprise water-soluble hyaluronan derivatives, preferably selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof.

If the hyaluronan or hyaluronan derivative are used in form of salts, the salt may be an alkali metal salt, preferably the sodium salt.

Nanofibers made from both hyaluronan and at least one hyaluronan derivative have the advantage that they can be tailored towards the specific needs of different applications via altering parameters such as rate of disintegration upon contact with aqueous solution, dissolving polar and/or hydrophobic components in water, covalent attachment of various amino compounds, cross-linking and encapsulation of various compounds, self-assembly, cavity formation and antiadhesion properties.

Examples of the above defined non-wovens or wovens made of water-soluble hyaluronan nanofibers are known in the art and have been propagated as scaffolds in tissue engineering or as layer in wound dressings. They are commercially available from the company Contipro.

Especially preferred water-soluble hyaluronan derivatives are sodium palmitoyl hyaluronate sodium caproyl hyaluronate, sodium palmitoyl formyl hyaluronate, sodium oleyl hyaluronate, sodium octanoyl formyl hyaluronate, sodium linolenoyl hyaluronate, sodium heptanoyl formyl hyaluronate, sodium caproyl formyl hyaluronate, sodium butanoyl hyaluronate, sodium anhydroformyl hyaluronate. This is the case as these derivatives are especially suited for preparing solid carriers for biologically active substances.

In a preferred embodiment the nanofibers comprise 40 to 95 wt.-% hyaluronan and 5 to 60 wt.-% of at least one water-soluble hyaluronan derivative.

Depending on the type and the degree of the employed hyaluronan derivatives the solubility of the nanofibers and hence of the solid carrier can be optimized for a given application.

Moreover, also the release profile for the active ingredient may be changed by altering the type of hyaluronan or the hyaluronan/ hyaluronan derivative ratio.

In a preferred embodiment the solid carrier constitutes a single-dose of the pharmaceutical composition.

Moreover, it is also preferred that the solid carrier is attached to a handle or tip. Such a handle or tip could be disposable or re-usable.

This is advantageous as in such a case the solid carrier of the pharmaceutical composition can be very small, because it is applied via the handle or tip, and hence does not need to be handled by the patients. Especially for patients with a tremor, such as elder patients, this is beneficial. Moreover, if the solid carrier is as small as possible any discomfort that might be experienced upon application as well as the disintegration time of the solid carrier are also minimized.

In various embodiments, the at least one therapeutically active agent is selected from the group consisting of prostaglandin and prostaglandin analogues, carbonic anhydrase inhibitors, beta-blockers, antibiotics, anti-histamines, alpha antagonists, antiseptics, anti-inflammatory drugs, and artificial tear formulations.

In a preferred embodiment the at least one therapeutically active agent is selected from the prostaglandin and prostaglandin analogues Latanoprost, Dorzolamid, Bimatoprost, Tafluprost, Travoprost and/or the carbonic anhydrase inhibitor Brinzolamid and/or the imidazolin Brimonidin and/or the beta-blocker Timolol and/or the antibiotics, Ofloxacin, Gentamicin, Dexamthason, Neomycin, and/or the anti-histamines Azelastin, Levocabastin, Ketotifen, and/or the alpha antagonist Tetryzolin and/or the antiseptic Bibrocathol and/or the anti-inflammatory drugs Prednisolon, Cromoglicic acid, Diclofenac, Ketorolac and/or Carbomer and Povidon.

In a preferred embodiment the active ingredient is applied to the nanofibers during their production and/or the at least one active ingredient is applied to the non-woven/woven during its production and/or the active ingredient is applied to the solid carrier after its production. The application may occur by impregnating the fibers, the non-woven/woven or the solid carrier with the active agent, for example in form of a powder or a solution, preferably a nonaqueous solution to prevent premature dissolution of the fibers.

While the present invention encompasses the above-described pharmaceutical compositions for ophthalmologic use as such, in various embodiments it is directed to the pharmaceutical composition as described above for use in the treatment or prevention of an ophthalmologic condition, such as an ophthalmologic disease or disorder. As used herein the term "ophthalmologic condition" refers to conditions, diseases or disorders of the eye ranging from discomfort experienced by a patient without any disease to serious and non-curable diseases of the eye, including all tissues, fluids and muscles making up the eye.

As used herein the terms "disorder" and "disease" refer to states that cause pain, dysfunction, distress, social problems to the person afflicted, or similar problems for those in contact with the person including injuries, disabilities, disorders, syndromes, infections and isolated symptoms.

In various embodiments, the ophthalmologic disease or disorder is selected from the group consisting of glaucoma, conjunctivitis, dry eye syndrome, macular degeneration, infection, inflammation and allergy.

Glaucoma is a term describing a group of ocular disorders that result in optic nerve damage, often associated with increased fluid pressure in the eye (intraocular pressure). The disorders can be roughly divided into two main categories, "open-angle" and "closed-angle glaucoma. Open-angle chronic glaucoma is painless, tends to develop slowly over time and often has no symptoms until the disease has progressed significantly. It is treated with either glaucoma medication to lower the pressure, or with various pressure-reducing glaucoma surgeries. Closed-angle glaucoma, however, is characterized by sudden eye pain, redness, nausea and vomiting, and other symptoms resulting from a sudden spike in intraocular pressure, and is treated as a medical emergency. Glaucoma can permanently damage vision in the affected eye(s), first by decreasing peripheral vision (reducing the visual field), and then potentially leading to blindness if left untreated. The many different subtypes of glaucoma can all be considered to be a type of optic neuropathy. The nerve damage involves loss of retinal ganglion cells in a characteristic pattern. Raised intraocular pressure (above 21 mmHg or 2.8 kPa) is the most important and only modifiable risk factor for glaucoma. Some may have high eye pressure for years and never develop damage, a condition known as "ocular hypertension". Conversely, the term 'low tension' or 'normal tension' glaucoma is used for those with optic nerve damage and associated visual field loss, but normal or low intraocular pressure.

Dry eye syndrome is an eye disease caused by eye dryness, which, in turn, is caused by either decreased tear production or increased tear film evaporation. It is the most common eye disease, affecting 5 - 6% of the human population.

Macular degeneration (diagnosis)(MDD), often age-related macular degeneration (AMD), is a medical condition that usually affects older adults and results in a loss of vision in the center of the visual field (the macula) because of damage to the retina. Macular degeneration can make it difficult or impossible to read or recognize faces, although enough peripheral vision remains to allow other activities of daily life. In the dry (nonexudative) form, cellular debris called drusen accumulates between the retina and the choroid, causing atrophy and scarring to the retina. In the wet (exudative) form, which is more severe, blood vessels grow up from the choroid behind the retina which can leak exudate and fluid and also cause hemorrhaging. It can be treated with laser coagulation, and more commonly with medication that stops and sometimes reverses the growth of blood vessels.

In addition, the invention also relates to the use of a non-woven or woven made of water-soluble fibers comprising or consisting of nanofibers made of hyaluronan or a pharmaceutically acceptable salt thereof as a solid carrier for at least one therapeutically active agent in an ophthalmologic pharmaceutical composition, wherein the non-woven or woven completely dissolves upon contact with the eye.

## Claims

1. Pharmaceutical composition for use in the treatment or prevention of ophthalmologic diseases or disorders, wherein the pharmaceutical composition comprises a solid carrier in form of a non-woven or woven made of water-soluble fibers impregnated with at least one therapeutically active agent, wherein the solid carrier dissolves upon contact with the eye.

2. Pharmaceutical composition for use according to claim 1, wherein the solid carrier dissolves upon contact with the eye within a time period of up to 10 seconds.

3. Pharmaceutical composition for use according to claim 1 or 2, wherein the fibers comprise or consist of hyaluronan (hyaluronic acid) or a pharmaceutically acceptable salt thereof.

4. Pharmaceutical composition for use according to any one of claims 1 to 3, wherein the water-soluble fibers comprise or consist of nanofibers.

5. Pharmaceutical composition for use according to any one of claims 1 to 4, wherein the solid carrier dissolves and the water-soluble fibers completely dissolve upon contact with the eye within a time period of up to 1 second.

6. Pharmaceutical composition for use according to any one of claims 1 to 5 wherein the fibers comprise 40 to 95 wt.-% hyaluronan and 5 to 60 wt.-% by weight of at least one water-soluble hyaluronan derivative selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof.

7. Pharmaceutical composition for use according to any one of claims 3 to 6, wherein the salt of hyaluronan or hyaluronan derivative is an alkali metal salt, preferably the sodium salt.

8. Pharmaceutical composition for use according to any one of claims 1 to 7, wherein the solid carrier has the form of a strip.

9. Pharmaceutical composition for use according to any one of claims 1 to 8, wherein the solid carrier is attached to a handle or tip.

10. Pharmaceutical composition for use according to any one of claims 1 to 9, wherein the at least one therapeutically active agent is selected from the group consisting of Latanoprost, Dorzolamid, Brinzolamid, Bimatoprost, Tafluprost, Travoprost, Brimonidin, Timolol, Ofloxacin, Gentamicin, Dexamthason, Neomycin, Azelastin, Prednisolon, Tetryzolin, Bibrocathol, Cromoglicinsäure, Levocabastin, Ketotifen, Diclofenac, Ketorolac, Carbomer, Povidon.

11. Pharmaceutical composition for use according to any one of claims 1 to 10, wherein the ophthalmologic disease or disorder is selected from the group consisting of glaucoma, dry eye syndrome, macular degeneration, infection, inflammation and allergy.

12. Ophthalmologic pharmaceutical composition comprising a solid carrier in form of a non-woven or woven made of water-soluble fibers impregnated with at least one therapeutically active agent, wherein the water-soluble fibers comprise or consist of nanofibers made of hyaluronan or a pharmaceutically acceptable salt thereof, wherein the solid carrier dissolves upon contact with the eye.

13. Ophthalmologic pharmaceutical composition according to claim 12, wherein the nanofibers comprise 40 to 95 wt.-% hyaluronan and 5 to 60 wt.-% by weight of at least one water-soluble hyaluronan derivative selected from the group consisting of propinylamino hyaluronan, azidyl hyaluronan, palmitoyl hyaluronan, formyl hyaluronan, caproyl hyaluronan, palmitoyl formyl hyaluronan, oleyl hyaluronan, octanoyl formyl hyaluronan, linolenoyl hyaluronan, heptanoyl formyl hyaluronan, caproyl formyl hyaluronan, butanoyl hyaluronan, anhydroformyl hyaluronan and pharmaceutically acceptable salts thereof.

14. Ophthalmologic pharmaceutical composition according to claim 12 or 13, wherein the salt of hyaluronan or hyaluronan derivative is an alkali metal salt, preferably the sodium salt.

15. Use of a non-woven or woven made of water-soluble fibers comprising or consisting of nanofibers made of hyaluronan or a pharmaceutically acceptable salt thereof as a solid carrier for at least one therapeutically active agent in an ophthalmologic pharmaceutical composition, wherein the non-woven or woven completely dissolves upon contact with the eye.
